# EUROPÄISCHE PATENTANMELDUNG

(11) **EP 1 669 346 A1**
(43) Veröffentlichungstag der Anmeldung: **14.06.2006**
(21) Anmeldenummer: 05025946.4
(22) Anmeldetag: 29.11.2005
(51) Int. Cl.: C07C 269/06, C07C 269/02, C07C 271/28, C07D 265/26

(54) **Verfahren zur Herstellung von Carbaminsäureester-Derivaten**

(30) Priorität: 10.12.2004 DE 102004059470
(71) Anmelder: LANXESS Deutschland GmbH, 51369 Leverkusen (DE)
(72) Erfinder: Hölzer, Bettina, Dr., 51379 Leverkusen (DE); Diehl, Herbert, Dr., 51373 Leverkusen (DE)

(57) **Zusammenfassung**

Bereitgestellt wird ein verbessertes Verfahren zur Herstellung von Carbaminsäureester-Derivaten der allgemeinen Formel (1) 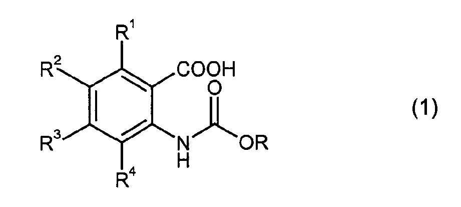 durch eine Umsetzung mit Kohlenmonoxid und Wasser in Gegenwart eines Palladiumkatalysators.

## Beschreibung

Die vorliegende Erfindung betrifft ein neues Verfahren zur Herstellung von Carbaminsäureester-Derivaten der allgemeinen Formel (1) 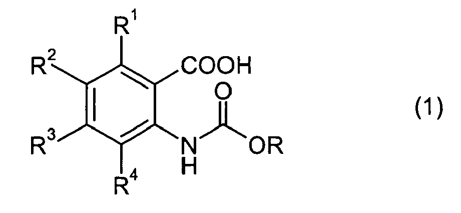 sowie die neue Verbindung (2-Bromo-4-methylphenyl)-carbaminsäure-hexadecylester (2") 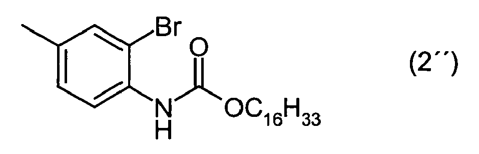 Carbaminsäureester-Derivate der allgemeinen Formel (1) und insbesondere (2-Carboxy-4-methylphenyl)-carbaminsäureester der allgemeinen Formel (1') 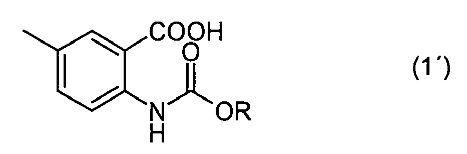 sind geeignete Zwischenprodukte für pharmazeutische Wirkstoffe.

So ist beispielsweise (2-Carboxy-4-methylphenyl)-carbaminsäurehexadecylester als Verbindung der Formel (1') mit R = C₁₆H₃₃ als ein Zwischenprodukt bei der Herstellung von 2-Hexadecyloxy-6-methyl-4*H*-3,1-benzoxazin-4-on der Formel (3) 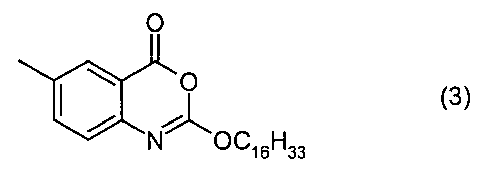 aus der ursprünglich veröffentlichten Fassung der WO-A-00/40569 bekannt. 2-Hexadecyloxy-6-methyl-4*H*-3,1-benzoxazin-4-on der Formel (3) ist darin als potentieller Wirkstoff zur Behandlung von Fettleibigkeit und Typ II Diabetes beschrieben. In dieser ursprünglich veröffentlichten Fassung der WO-A-00/40569 werden für die Herstellung von 2-Hexadecyloxy-6-methyl-4*H*-3,1-benzoxazin-4-on (3) zwei Synthesewege 1 und 2 beschrieben, bei denen man jeweils von der 5-methylsubstituierten Anthranilsäure (4) ausgeht.

Im zweistufigen Syntheseweg 1 wird die 5-methylsubstituierte Anthranilsäure (4) mit Hexadecylchlorformiat (5) und anschließend mit Methylchlorformiat zu 2-Hexadecyloxy-6-methyl-4*H*-3,1-benzoxazin-4-on (3) umgesetzt, wobei allerdings nur eine Gesamtausbeute von 31 % erzielt wird.

Der einstufige Syntheseweg 2 mit einem Überschuß an Pyridin liefert 2-Hexadecyloxy-6-methyl-4*H-3,*1-benzoxazin-4-on (3) in einer noch geringeren Ausbeute von 15 %.

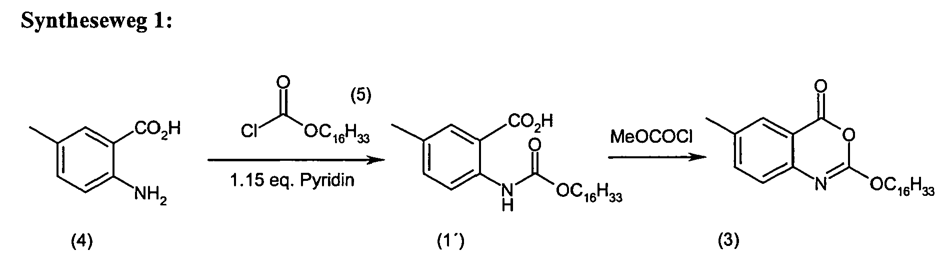

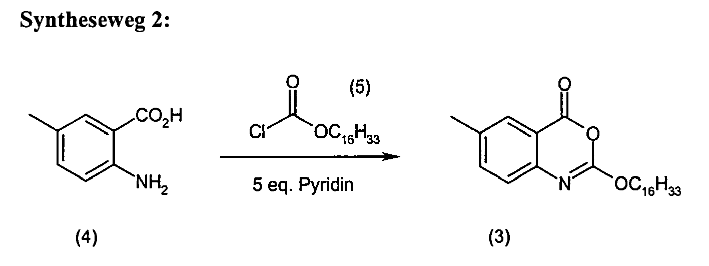

Die für beide Synthesewege 1 und 2 benötigte Ausgangsverbindung, die 5-methylsubstituierte Anthranilsäure (4), ist jedoch nicht leicht zugänglich.

Sie wird nach der in. J. Org. Chem. 1952, 17, 141 beschriebenen Methode hergestellt: Dabei geht man von p-Toluidin aus, welches mit Chloralhydrat und Hydroxylamin-Hydrochlorid umgesetzt wird. Das erhaltenen Oxim wird säurekatalysiert cyclisiert und anschließend der Ring wieder oxidativ unter basischen Bedingungen gespalten.

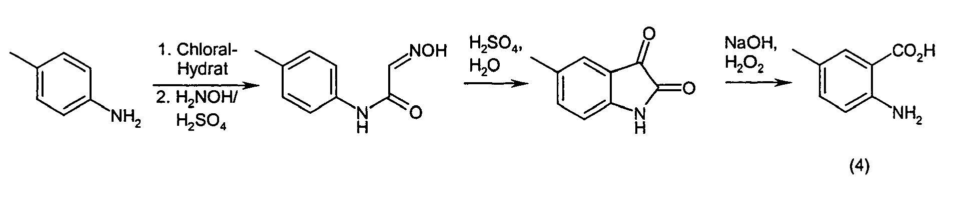

Nachteilig an dieser Synthese sind die niedrigen Ausbeuten und die Tatsache, dass nur mit sehr geringen Konzentrationen gearbeitet werden kann. Aus diesem Grund ist dieser Syntheseweg für eine technische Umsetzung nicht attraktiv.

Alternativ gibt es folgende weitere, prinzipiell bekannte Zugangswege zu Anthranilsäuren:
- Aus J. Org. Chem. 1978, 43, 220 und Chem. Ber. 1909, 42, 430 ist es bekannt, ausgehend von 3-Cyanotoluol zunächst eine Nitrierung durchzuführen, dann die Nitrogruppe zu reduzieren und anschließend das Nitril zur Carbonsäure zu hydrolysieren. 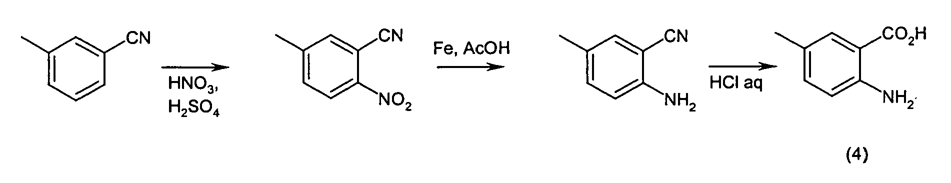 Nachteilig an dieser Synthese ist, dass die Nitrierung von 3-Cyanotoluol nicht selektiv verläuft und deshalb ein weiterer Reinigungsschritt notwendig ist. Dies erfordert zusätzlichen Aufwand und vermindert die Ausbeute.
- Denselben Nachteil hat auch die in J. Chem. Soc. Perkin 1, 1973, 2940 beschriebene Synthese ausgehend von 3-Tolylsäure mit anschließender Nitrierung und Reduktion der Nitrogruppe.
- Die aus Monatsh. Chem. 1920, 41, 155 bekannte Synthese ausgehend von 2,4-Dimethyl-1-nitrobenzol ist ebenfalls wenig geeignet, da die Oxidation der Methylgruppe in Nachbarschaft zur Nitrogruppe unselektiv verläuft und daher eine aufwendige Isomerentrennung notwendig ist. 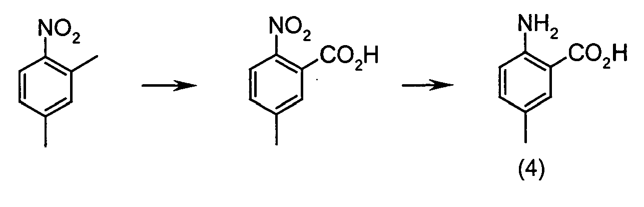
- Aus der EP-A-0 034 292 ist ein Verfahren zur Herstellung von ggf. substituierten Anthranilsäuren bekannt, welches eine übergangsmetallkatalysierte Carbonylierungs-reaktion mit Kohlenmonoxid zu einem Anthranilsäurederivat umfasst. Diese Carbonylierungsreaktion findet in einem wässrigen Reaktionsmedium statt, welches ein Trialkylamin und einen Katalysator, gebildet aus Palladium und einem tertiären Phosphin, enthält. Die Anthranilsäurederivate können durch Abspaltung der Schutzgruppe erhalten werden. Die für die Carbonylierung eingesetzten Edukte werden ausgehend von ggf. substituierten Anilinen erhalten wie im nachfolgenden Reaktionsschema prinzipiell gezeigt: 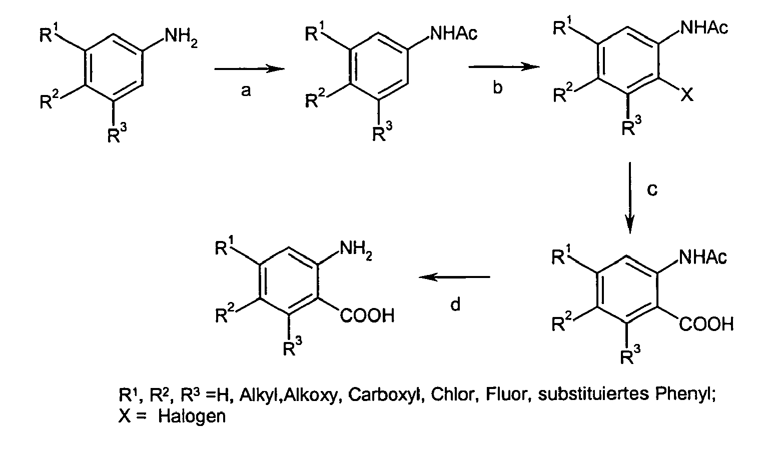 In der EP-A-0 034 292 wird beschrieben, dass diese Reaktionssequenz aus Acetylierung (a), Halogenierung (b), Carbonylierung (c) und anschließende Abspaltung der Acetylgruppe (d) die ggf. substituierten Anthranilsäuren in guten Ausbeuten (>80 %) liefert. Nachteilig ist jedoch die Einführung der Acetylgruppe. Dies ist notwendig, da freie Aniline bei übergangsmetallkatalysierten Carbonylierungsreaktionen aufgrund ausgeprägter Komplexierung nur schlechte Ausbeuten ergeben. [J. Org. Chem.1981, 46, 4614-4617].
- Aus der WO-A-97/28118 ist ein vergleichbares Verfahren bekannt.

Aufgrund der zuvor beschriebenen, diversen Schwierigkeiten bei den bekannten Herstellverfahren für ggf. substituierte Anthranilsäuren und den nur unbefriedigenden und damit für das Gesamtverfahren limitierenden Ausbeuten der sich anschließenden Synthesewege 1 und 2 bestand die Aufgabe der vorliegenden Erfindung darin, ein verbessertes Verfahren zur Herstellung von Carbaminsäureester-Derivaten der allgemeinen Formel (1) zur Verfügung zu stellen.

Gegenstand der vorliegenden Erfindung ist ein Verfahren zur Herstellung von Carbaminsäureester-Derivaten der allgemeinen Formel (1) 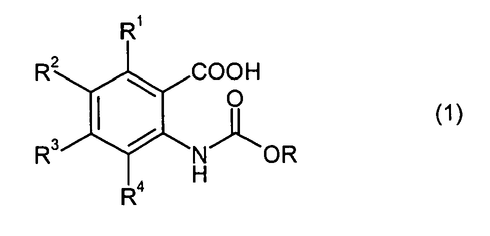 worin
- R¹, R², R³, R⁴: gleich oder verschieden sind und Wasserstoff, einen Alkyl-, Alkoxy-, Carboxyl-, Chlor-, Fluor- oder gegebenenfalls durch Alkyl- oder Alkoxy-Reste substituierten Phenyl-Rest bedeuten,
und
- R: für einen Alkyl, Aryl oder Heteroaryl-Rest steht
durch Umsetzung von Verbindungen der allgemeinen Formel (2) 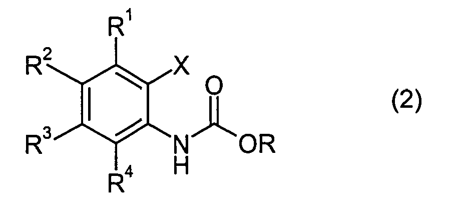 wobei
- X: für Brom oder Jod steht und
R¹, R², R³, R⁴ sowie R die für die allgemeine Formel (1) genannten Bedeutungen besitzen,
mit Kohlenmonoxid und Wasser in Gegenwart eines Palladiumkatalysators.

Dass die Herstellung der Carbaminsäureester-Derivate der allgemeinen Formel (1) durch die erfmdungsgemäße Carbonylierung gelingt, war ausgehend vom Stand der Technik überraschend und unvorhersehbar.

Die Carbonylierung einer aromatischen Halogenverbindung mit einer Carbamat-Seitenkette, insbesondere in der ortho-Position zum Halogen-Rest und mit einer freien NH-Gruppe ist literaturunbekannt, und es ist überraschend, dass diese Carbonylierung mit exzellenten Ergebnissen gelingt, da die Schwierigkeiten bei Palladium-katalysierten Carbonylierungsreaktionen von 2-Haloanilinen hinlänglich bekannt sind und eine Carbamat-Gruppe, so wie in Verbindung (2) vorhanden, stärker als eine Acetyl-Gruppe komplexierend wirkt.

Bei den im erfindungsgemäßen Verfahren eingesetzten Verbindungen der allgemeinen Formel (2) sowie den entsprechenden erhaltenen Verbindungen der allgemeinen Formel (1) haben die aufgeführten Substituenten folgenden Bedeutungen:

Alkyl steht im Rahmen dieser Erfindung für einen geradkettigen oder verzweigten Alkylrest mit 1 bis 22 Kohlenstoffatomen wie Methyl, Ethyl, sowie die geradkettigen oder sämtlichen verzweigten Reste von Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl oder Docosyl-Reste.

Bei den Resten R¹, R², R³ und R⁴ steht Alkyl bevorzugt für einen geradkettigen oder verzweigten Alkylrest mit 1 bis 7 Kohlenstoff-Atomen, besonders bevorzugt für Methyl, Ethyl, n-Propyl, isoPropyl, n-Butyl, iso-Butyl oder tert.Butyl.

Für den Rest R steht Alkyl bevorzugt für einen geradkettigen oder verzweigten Alkylrest mit 16-22 Kohlenstoffatomen.

Alkoxy steht im Rahmen dieser Erfmdung bevorzugt für einen geradkettigen oder verzweigten Alkoxyrest mit 1 bis 7 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielsweise und vorzugsweise seien genannt: Methoxy, Ethoxy, n-Propoxy, Isopropoxy, n-Butoxy, iso-Butoxy, tert.Butoxy, n-Pentoxy, n-Hexoxy und n-Heptoxy.

Heteroaryl steht im Rahmen dieser Erfindung bevorzugt für einen aromatischen, mono- oder bicyclischen Rest mit 5 bis 10 Ringatomen und bis zu 5 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff- oder Heteroatom gebunden sein. Beispielsweise und vorzugsweise seien genannt: Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl und Isochinolinyl.

In einer besonders bevorzugten Variante des erfindungsgemäßen Verfahrens wird eine Verbindung der allgemeinen Formel (2') 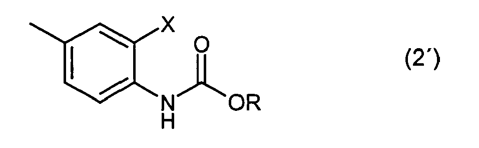 worin
- X: für Chlor oder Brom, insbesondere Brom und
- R: für einen geradkettigen oder verzweigten Alkylrest mit 16-22 Kohlenstoffatomen, bevorzugt für einen geradkettigen Alkylrest mit 16 Kohlenstoffatomen steht
mit Kohlenmonoxid und Wasser in Gegenwart eines Palladiumkatalysators zu einem Carbaminsäureester-Derivat der allgemeinen Formel (1') umgesetzt, 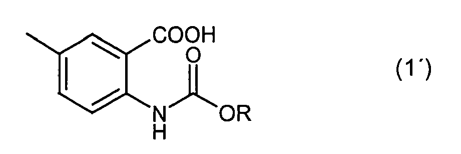 wobei
- R: die für die allgemeine Formel (2') genannte Bedeutung besitzt.

Als Palladiumkatalysatoren können im erfindungsgemäßen Verfahren zur Herstellung der Carbaminsäureester-Derivate der allgemeinen Formel (1) z. B. solche des Typs X₂Pd(PPh₃)₂ eingesetzt werden, wobei Ph einen gegebenenfalls substituierten Phenylrest darstellt und X für Halogen, bevorzugt Chlor oder Brom, steht. Diese Palladiumkatalysatoren kann man entweder als solche dem Reaktionsgemisch zusetzen oder aber günstigerweise auch in situ aus PdX₂ und PPh₃ herstellen. Die Komponente PPh₃ kann dabei auch im Überschuß eingesetzt werden. Bezogen auf die Verbindung der allgemeinen Formel (2) setzt man beispielsweise 0.1 bis 1 Mol-%, bevorzugt 0,1 bis 0,5 Mol-% Palladiumkatalysator ein.

Das erfindungsgemäße Verfahren wird üblicherweise in Gegenwart einer Base durchgeführt. Als Base können beispielsweise primäre, sekundäre und tertiäre Amine, Acetate, Carbonate und Hydrogencarbonate eingesetzt werden. Bevorzugt sind Carbonate und Hydrogencarbonate. Bezogen auf 1 Mol der Verbindung der allgemeinen Formel (2) kann man beispielsweise 0.9 bis 5 mol, vorzugsweise 1.0 bis 2.0 Mol Base einsetzen.

Die Reaktionstemperatur ist nicht kritisch: Üblicherweise wird das erfmdungsgemäße Verfahren bei einer Reaktionstemperatur im Bereich von 60 bis 120°C, bevorzugt im Bereich von 80 bis 115°C durchgeführt. Der Reaktionsdruck liegt üblicherweise bei 2 bis 30 bar, bevorzugt bei 2-15 bar.

Üblicherweise wird das erfindungsgemäße Verfahren so durchgeführt, dass man die Verbindung der allgemeinen Formel (2) zusammen dem Palladium-Katalysator als solchem oder dessen Vorprodukte PdX₂ und PPh₃ in einem Druckgefäß vorlegt, danach Wasser zufügt sowie gegebenenfalls die Base. Anschließend wird auf 60 bis 120°C erhitzt und 2 bis 30 bar Kohlenmonoxid zudosiert und dieser Druck aufrechterhalten, bis kein Kohlenmonoxid mehr aufgenommen wird.

Gegebenenfalls kann zur Verbesserung der Löslichkeit ein Gemisch aus einem oder mehreren Lösungsmitteln mit Wasser verwendet werden. Bevorzugte zusätzliche Lösungsmittel sind Nitrile wie Acetonitril, Amide wie Dimethylformamid, Ether wie Dioxan und Tetrahydrofuran und verzweigte, höhere Alkohole, die nicht als Nucleophil mit dem Wasser konkurrieren.

Die Herstellung der Verbindungen der allgemeinen Formel (2) 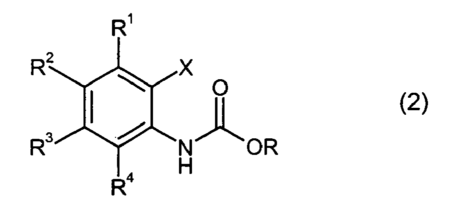 ist auf zwei verschiedenen Syntheserouten möglich, die beide von einem gegebenenfalls substituierten Anilin ausgehen.

In der Syntheseroute A werden die Verbindungen der allgemeinen Formel (2) hergestellt, indem man ein gegebenenfalls substituiertes Anilin der allgemeinen Formel (6) 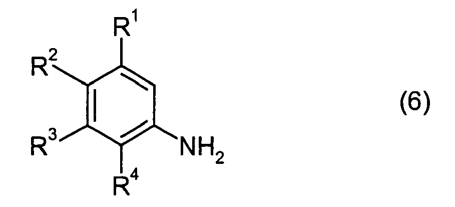 entweder
- Ia): mit Phosgen oder einer in-situ Phosgen erzeugenden oder Phosgen enthaltenden Substanz umsetzt und
- Ib): die dabei erhaltene Verbindung der allgemeinen Formel (7) 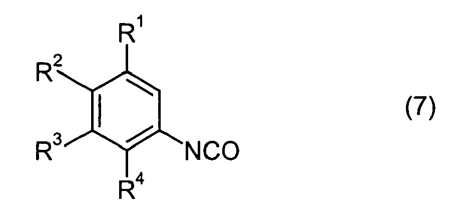
mit einem Alkohol der Formel ROH umsetzt
oder
- II): mit einer Verbindung der Formel Cl-C(=O)OR umsetzt
und die sowohl nach Schritt (Ia) und (Ib) bzw. dem alternativen Schritt (II) erhaltene Verbindung der allgemeinen Formel (8) 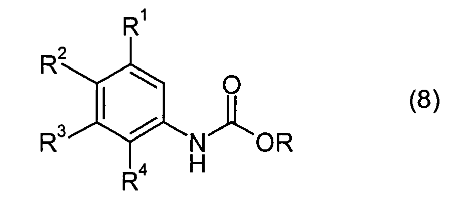 einer Halogenierung unter Bildung einer Verbindung der allgemeinen Formel (2) unterzieht, wobei in allen vorgenannten Formeln die Reste R¹, R², R³, R⁴ sowie R die für die allgemeine Formel (1) genannten Bedeutungen besitzen.

Die Syntheseroute A kann auch wie folgt schematisch dargestellt werden:

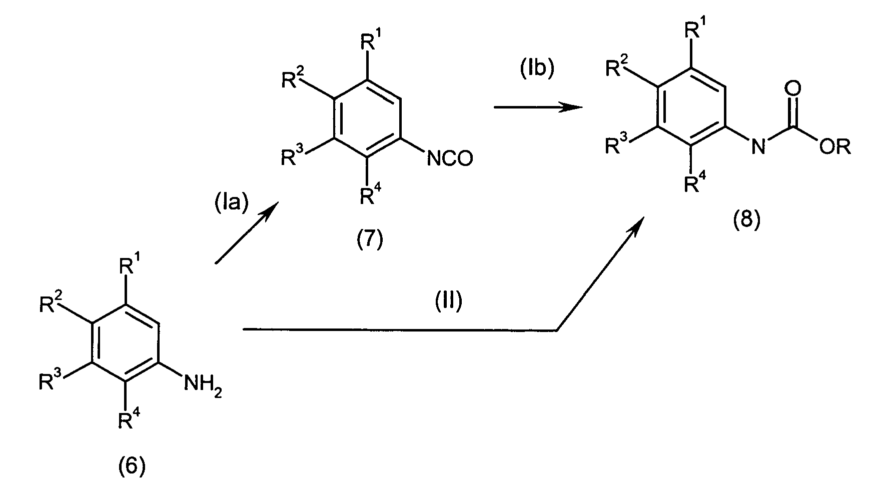

Die Durchführung der Schritte (Ia) und (Ib) erfolgt unter analoger Anwendung der in Chem.Ber. 1888, 21, 411 bzw. Bull. Soc. Chim.Fr. 1904, 31, 50 enthaltenen Offenbarung.

Als Phosgen in-situ erzeugende Substanz kann beispielsweise Trichlormethylchlorformiat (Diphosgen) eingesetzt werden. Als Phosgen enthaltende Substanz kann z.B. Oxalylchlorid eingesetzt werden, welches in technischer Qualität häufig kleinere Mengen Phosgen enthält.

Die Durchführung des Schrittes (II) erfolgt unter analoger Anwendung der aus Chem. Ber. 1870, 3, 655 bekannten Offenbarung.

Die sich anschließende Halogenierung wird nach dem Fachmann bekannten Methoden durchgeführt. Die bevorzugte Bromierung der Verbindungen der allgemeinen Formel (9) kann unter Ein-satz von Br₂/HOAc oder Br₂/H₂O₂/HOAc ebenfalls nach dem Fachmann bekannten Methoden durchgeführt werden.

In der Syntheseroute B werden die Verbindungen der allgemeinen Formel (2) hergestellt, indem man ein gegebenenfalls substituiertes Anilin der allgemeinen Formel (6) 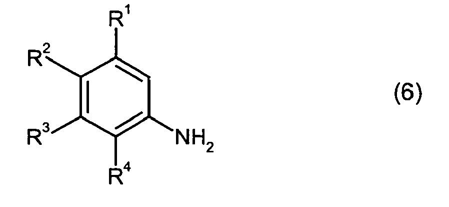
- 1): einer Halogenierung unterzieht und
- 2): die dabei erhaltene Verbindung der allgemeinen Formel (9) 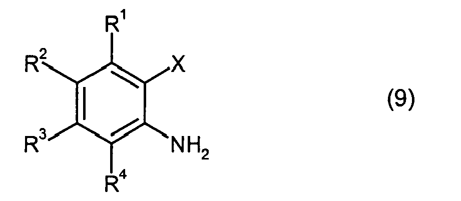
entweder
- IIIa): mit Phosgen oder mit einer in-situ Phosgen erzeugenden oder Phosgen enthaltenden Substanz umsetzt und
- IIIb): die dabei erhaltene Verbindung der Formel (10) 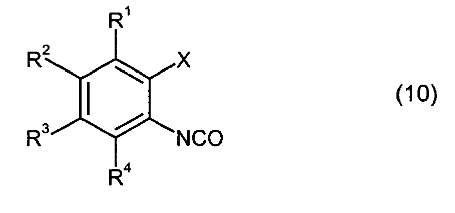
mit einem Alkohol der Formel ROH umsetzt
oder
- IV): in einem einzigen Schritt mit Cl-C(=O)OR umsetzt,
wobei in allen vorgenannten Formeln die Reste R¹, R², R³, R⁴ sowie R die für die allgemeine Formel (1) genannten Bedeutungen besitzen.

Die Halogenierung gemäß Syntheseroute B, Schritt 1 wird analog zu Organic Syntheses, Coll. Vol. 1, S. 111 durchgeführt.

Die Umsetzung gemäß Syntheseroute B, Schritt IIIa wird analog zu Bioorg. Med. Chem. 1999, 7, 1597 durchgeführt. Als Phosgen in-situ erzeugende Substanz kann beispielsweise Trichlormethylchlorformiat (Diphosgen) eingesetzt werden. Als Phosgen enthaltende Substanz kann z.B. Oxalylchlorid eingesetzt werden, welches in technischer Qualität häufig kleinere Mengen Phosgen enthält.

DieUmsetzung gemäß Syntheseroute B, Schritt IIIb wird analog zu der in Bull. Soc. Chim. Fr. 1904, 31, 50 enthaltenen Offenbarung durchgeführt.

Die Umsetzung gemäß Syntheseroute B, Schritt IV wird analog zu der in Chem. Ber. 1870, 3, 655 enthaltenen Offenbarung durchgeführt.

Gegenstand der vorliegenden Erfindung ist ferner die Verbindung der Formel (2")

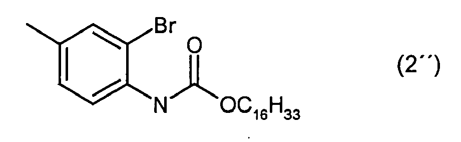

Diese Verbindung ist von Bedeutung, da durch sie der spezielle Zugang zum 2-Hexadecyloxy-6-methyl-4*H*-3,1-benzoxazin-4-on der Formel (3) mit den beschriebenen Syntheserouten in deutlich einfacherer Form möglich ist als über die bisherigen Wege unter Durchlaufen der Zwischenstufe des (2-Carboxy-4-methylphenyl)-carbaminsäure-hexadecylesters. Diese Wege verlaufen ohne Bildung von Fehlisomeren, d.h. es sind somit keine aufwändigen Reinigungs- oder Kristallisationsschritte nötig.

Die vorliegende Erfindung ermöglicht es ferner, 2-Alkyloxy-6-methyl-4*H*-3,1-benzoxazin-4-one der allgemeinen Formel (11) herzustellen, 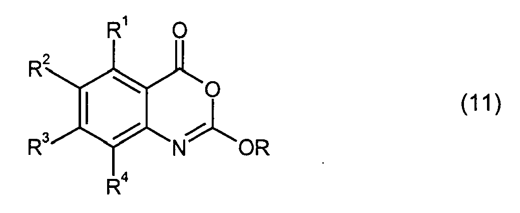 durch Umsetzung von Verbindungen der allgemeinen Formel (2) 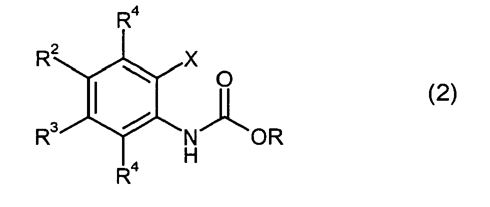 mit Kohlenmonoxid und Wasser in Gegenwart eines Palladiumkatalysators
unter Bildung von Carbaminsäureester-Derivaten der allgemeinen Formel (1) 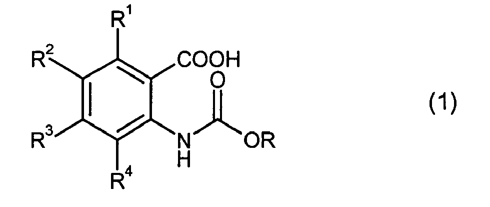 und deren Umsetzung mit Methyl- oder Ethylchlorformiat,
wobei R¹, R², R³, R⁴ sowie R in allen vorgenannten Formeln immer die für die allgemeine Formel (1) genannten Bedeutungen besitzen.

Die vorgenannte Umsetzung mit Methyl- oder Ethylchlorformiat erfolgt nach dem Fachmann geläufigen Methoden.

Der Vorteil dieses gesamten Verfahrens besteht darin, dass das zusätzliche Einführen und Abspalten der Acetylgruppe als Schutzgruppe für die Carbonylierung vermieden werden kann. Stattdessen wird direkt die 2-Halogen-Verbindung der allgemeinen Formel (2) in die Carbonylierungsreaktion eingesetzt. Durch den Wegfall der Abspaltung der Acetylgruppe wird eine kürzere und damit wirtschaftlich attraktivere Synthese möglich.

Ein weiterer Vorteil dieser Syntheseroute liegt insbesondere bei der Herstellung von (2-Bromo-4-methylphenyl)-carbaminsäure-hexadecylester (2") darin, dass unter Berücksichtigung der vorgeschaltenen Synthesewege wie oben beschrieben ausgehend von p-Toluidin, welches isomerenrein kommerziell verfügbar ist, kein Isomerenproblem wie bei einer Route ausgehend von 3-Cyanotoluol oder 3-Tolylsäure besteht.

### Beispiele:

### Beispiel 1:

### Synthese von 4-Methylphenyl-carbaminsäure-hexadecylester

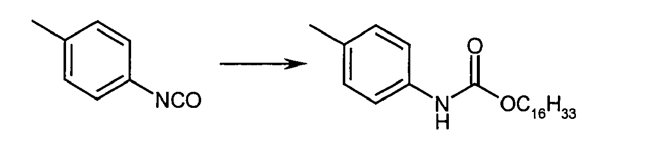

Zu einer Lösung von 50 g (375 mmol) p-Tolyl-isocyanat in 50 ml Toluol werden 91 g (375 mmol) 1-Hexadecanol gegeben und die entstandene Lösung unter Rückfluß 8 h erhitzt. Nach Abkühlen auf Raumtemperatur läßt man 12 h bei dieser Temperatur nachrühren und filtriert anschließend den ausgefallenen Feststoff ab. Der farblose Feststoff wird zweimal mit je 10 ml Toluol gewaschen und anschließend im Vakuum getrocknet. Man erhält 80 g (213 mmol, 57 %) des gewünschten Carbamats in Form eines farblosen Feststoffs mit einem Schmelzpunkt von 75°C. Der Schmelzpunkt stimmt mit den Literaturdaten überein (75-76°C, Microchem. J. 1962, 6, 179)

¹H-NMR (CDCl₃, 400 MHz): δ = 0.88 ppm (t, J = 7.3 Hz, 3 H), 1.25-1.40 (m, 26 H), 1.66 (sext, J = 6.9 Hz, 2 H), 2.30 (s, 3 H), 4.14 (t, J = 6.9 Hz, 2 H), 6.53 (br, 1 H), 7.10 (d, J = 7.8 Hz, 2 H), 7.25 (d, J = 8.3 Hz, 2 H).

Die Elementaranalyse ergibt:
Berechnet: C 76.8 %, H 11.0 %, N 3.7 %
Gefunden: C 76.9 %, H 11.2 %, N 3.7 %.

### Beispiel 2:

### Synthese von (2-Bromo-4-methylphenyl)-carbaminsäure-hexadecylester

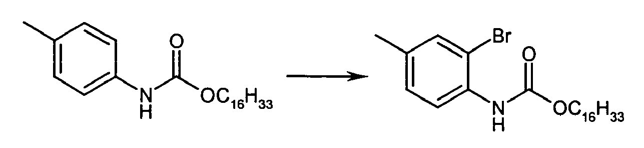

Zu einer Lösung von 45 g (119 mmol) des Carbamats in 225 ml (235 g) Eisessig wird bei Raumtemperatur innerhalb von 1 h 19 g (119 mmol) Brom zugetropft und anschließend die entstandene Lösung 1 h bei Raumtemperatur nachgerührt. Nach Zusetzen von weiteren 25 ml (26 g, 437 mmol) Eisessig läßt man das Reaktionsgemisch 5 h bei 40°C rühren und anschließend auf Raumtemperatur abkühlen. Der ausgefallene Feststoff wird abfiltiert und mit 20 ml Eisessig gewaschen. Nach Trocknen im Vakuum erhält man 40 g (88 mmol, 74 %) der gewünschten Bromverbindung in Form eines farblosen Feststoffs mit einem Schmelzpunkt von 57°C.

¹H-NMR (CDCl₃, 400 MHz): δ = 0.93 ppm (t, J = 6.6 Hz, 3 H), 1.25-1.43 (m, 26 H), 1.73 (sext, J = 6.8 Hz, 2 H), 2.33 (s, 3 H), 4.21 (t, J = 6.7 Hz, 2 H), 7.04 (br, 1 H), 7.14 (d, J = 8.4 Hz, 1 H), 7.37 (s, 1 H), 8.02 (d, J = 8.3 Hz, 1 H).

¹³C-NMR (CDCl₃, 100 MHz): δ = 14.2 ppm, 20.4, 22.7. 25.9, 29.0, 29.3, 29.4, 29.6 (2C), 29.7 (2C), 29.8 (4C), 32.0, 65.7, 112.5, 120.3, 129.0, 132.5, 133.5, 134.1, 153.5.

Die Elementaranalyse ergibt:
Berechnet: C 63.4 %, H 8.9 %, N 3.1 %
Gefunden: C 63.6 %, H 8.9 %, N 3.1 %.

### Beispiel 3:

### Synthese von 2-Hexadecyloxycarbonyl-amino-5-methyl-benzoesäure

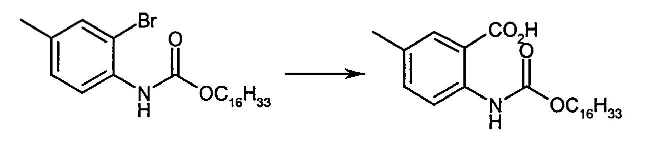

In einem Autoklaven werden 217.5 g (478.5 mmol) (2-Bromo-4-methyl-phenyl)-carbaminsäure-hexadecylester, 0.5 g (0.7 mmol) Bis(triphenylphosphin)-palladiumdichlorid und 2.5 g (9.3 mmol) Triphenylphosphin vorgelegt. Der Autoklav wird verschlossen, mit Stickstoff gespült und eine sauerstofffreie Lösung von 78.1 g (565.3 mmol) Kaliumcarbonat in 400 ml Wasser hinzugefügt. Der Autoklav wird evakuiert, dann werden 2 bar Kohlenmonoxid aufgedrückt und auf 115°C erhitzt. Der Druck wird anschließend auf 8 bar eingestellt. Nach beendeter CO-Aufnahme wird auf RT abgekühlt, und es werden 200 ml Toluol zugegeben. Der pH-Wert wird mit 2M wässriger HCl-Lösung auf 2 eingestellt und die organische Phase abgetrennt. Die wässrige Phase wird erneut mit 100 ml Toluol extrahiert, die organische Phase abgetrennt und die beiden Toluol-Extrakte vereinigt. Nach Entfernen des Lösungsmittels im Vakuum werden 154.9 g (369.2 mmol, 77 %) 2-Hexadecyloxy-carbonyl-amino-5-methyl-benzoesäure in Form eines schwach gelb gefärbten Feststoffs erhalten.

¹H-NMR (CDCl₃, 400 MHz): δ = 0.88 ppm (t, J = 6.7 Hz, 3 H), 1.24-1.40 (m, 26 H), 1.70 (sext, J = 6.8 Hz, 2 H), 2.33 (s, 3 H), 4.17 (t, J = 6.8 Hz, 2 H), 7.38 (d, J = 8.7 Hz, 1 H), 7.90 (s, 1 H), 8.35 (d, J = 8.6 Hz, 1 H). Signal des NH-Protons nicht erkennbar.

¹³C-NMR (CDCl₃, 100 MHz): δ = 14.1 ppm, 20.5, 22.7. 25.9, 29.0, 29.3, 29.4, 29.6 (2 C), 29.7 (6 C), 32.0, 65.5, 113.6, 119.0, 131.1, 131.8, 136.3, 140.1, 153.9, 172.5.

### Beispiel 4:

### Synthese von 2-Hexadecyloxy-6-methyl-4H-3,1-benzoxazin-4-on

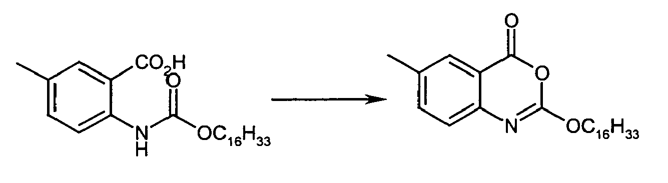

4.0 g (10.0 mmol) 2-Hexadecyloxycarbonyl-amino-5-methyl-benzoesäure und 20 ml Pyridin werden bei 0°C unter Stickstoff-Atmosphäre vorgelegt und zu der entstandenen Lösung bei 0°C innerhalb von 20 min 4.93 g (45.4 mmol) Ethylchloroformiat zugetropft. Nach einer Nachrührzeit von 1 h bei 0°C und 2 h bei Raumtemperatur gibt man die Reaktionsmischung in 30 ml Eiswasser. Der Feststoff wird abfiltiert und im Vakuum getrocknet. Man erhält 3.3 g (8.2 mmol, 82 %) 2-Hexadecyloxy-6-methyl-4*H*-3,1-benzoxazin-4-on in Form eines schwach gelb gefärbten Feststoffs mit einem Schmelzpunkt von 67°C (Literatur: 72-73°C, WO 00/40569).

¹H-NMR (CDCl₃, 400 MHz): δ = 0.86 ppm (t, J = 6.6 Hz, 3 H), 1.24-1.42 (m, 26 H), 1.75-1.82 (m, 2H), 2.40 (s, 3 H), 4.41 (t, J = 6.8 Hz, 2 H), 7.30 (d, J = 8.3 Hz, 1 H), 7.51 (dd, J = 8.2, 1.9 Hz, 1 H), 7.90 (d, J = 0.9 Hz, 1 H).

Die ¹H-NMR-Daten stimmen mit den Literaturdaten aus der WO-A-00/40569 überein.

## Patentansprüche

1. Verfahren zur Herstellung von Carbaminsäureester-Derivaten der allgemeinen Formel (1) 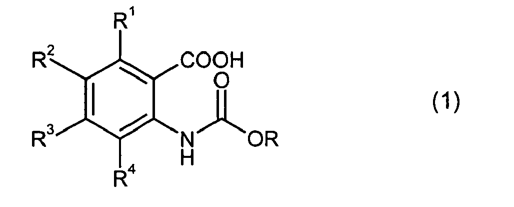 worin
R¹, R², R³, R⁴ gleich oder verschieden sind und Wasserstoff, einen Alkyl-, Alkoxy-, Carboxyl-, Chlor-, Fluor- oder gegebenenfalls durch Alkyl- oder Alkoxy-Reste substituierten Phenyl-Rest bedeuten,
und
R für einen Alkyl, Aryl oder Heteroaryl-Rest steht
durch Umsetzung von Verbindungen der allgemeinen Formel (2) 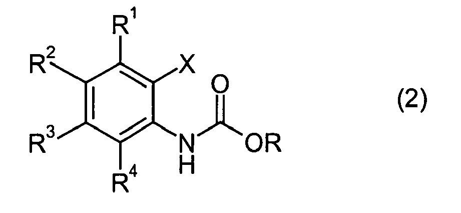 wobei
X für Brom oder Jod steht und
R¹, R², R³, R⁴ sowie R die für die allgemeine Formel (1) genannten Bedeutungen besitzen,
mit Kohlenmonoxid und Wasser in Gegenwart eines Palladiumkatalysators.

2. Verfahren nach Anspruch 1, **dadurch gekennzeichnet, dass** Verbindungen der allgemeinen Formel (2'), 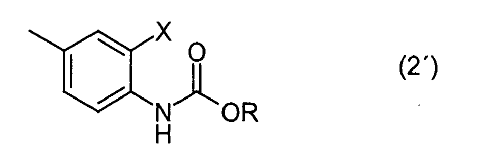 worin
X für Chlor oder Brom, insbesondere Brom und
R für einen geradkettigen oder verzweigten Alkylrest mit 16-22 Kohlenstoffatomen, bevorzugt für einen geradkettigen Alkylrest mit 16 Kohlenstoffatomen steht
mit Kohlenmonoxid und Wasser in Gegenwart eines Palladiumkatalysators unter Bildung eines Carbaminsäureester-Derivats der allgemeinen Formel (1') eingesetzt werden, 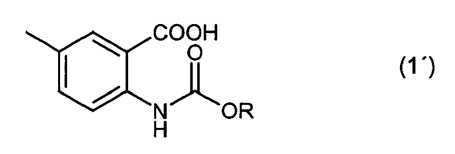 wobei R die für die allgemeine Formel (2') genannte Bedeutung besitzt.

3. Verfahren nach Anspruch 1 oder 2, **dadurch gekennzeichnet, dass** als Palladiumkatalysatoren solche des Typs X₂Pd(PPh₃)₂ eingesetzt werden, wobei Ph einen gegebenenfalls substituierten Phenylrest darstellt und X für Halogen, bevorzugt Chlor oder Brom, steht.

4. Verfahren nach einem oder mehreren der Ansprüche 1 bis 3, **dadurch gekennzeichnet, dass** das Verfahren in Gegenwart einer Base durchgeführt wird, vorzugsweise in Gegenwart von primären, sekundären oder tertiären Aminen, Acetaten, Carbonaten oder Hydrogencarbonaten.

5. Verfahren nach einem oder mehreren der Ansprüche 1-4, **dadurch gekennzeichnet, dass** die Herstellung der Verbindungen der allgemeinen Formel (2) 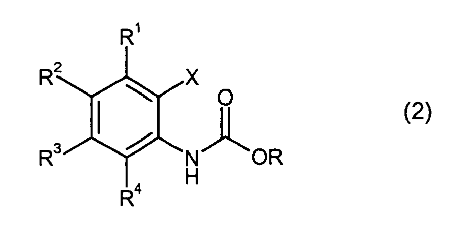 erfolgt, indem man ein gegebenenfalls substituiertes Anilin der allgemeinen Formel (6) 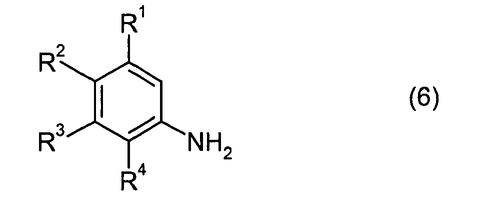 entweder
Ia) mit Phosgen oder einer in-situ Phosgen erzeugenden oder Phosgen enthaltenden Substanz umsetzt und
Ib) die dabei erhaltene Verbindung der allgemeinen Formel (7) 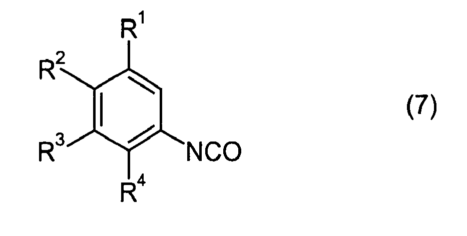
mit einem Alkohol der Formel ROH umsetzt
oder
II) mit einer Verbindung der Formel Cl-C(=O)OR umsetzt
und die sowohl nach Schritt (Ia) und (Ib) bzw. dem alternativen Schritt (II) erhaltene Verbindung der allgemeinen Formel (8) 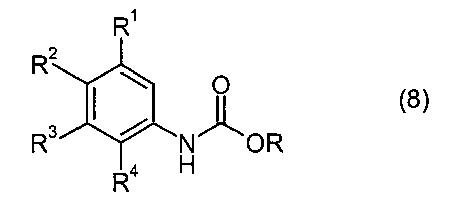 einer Halogenierung unter Bildung einer Verbindung der allgemeinen Formel (2) unterzieht, wobei in allen vorgenannten Formeln die Reste R¹, R², R³, R⁴ sowie R die für die allgemeine Formel (1) genannten Bedeutungen besitzen.

6. Verfahren nach einem oder mehreren der Ansprüche 1-4, **dadurch gekennzeichnet, dass** die Herstellung der Verbindungen der allgemeinen Formel (2) erfolgt, indem man ein gegebenenfalls substituiertes Anilin der allgemeinen Formel (6) 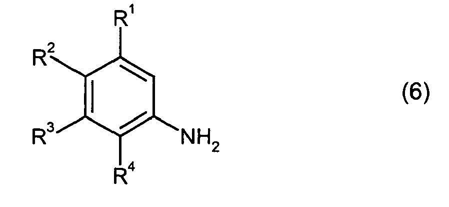
1) einer Halogenierung unterzieht und
2) die dabei erhaltene Verbindung der allgemeinen Formel (9) 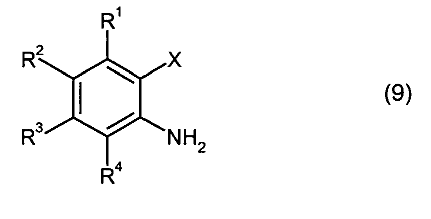 entweder
IIIa) mit Phosgen oder einer in-situ Phosgen erzeugenden oder Phosgen enthaltenden Substanz umsetzt und
IIIb) die dabei erhaltene Verbindung der Formel (10) 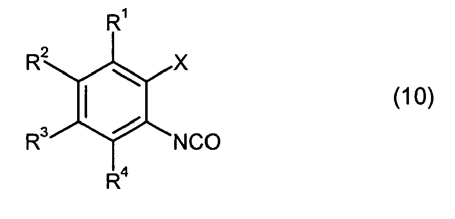 mit einem Alkohol der Formel ROH umsetzt
oder
IV) in einem einzigen Schritt mit Cl-C(=O)OR umsetzt,
wobei in allen vorgenannten Formeln die Reste R¹, R², R³, R⁴ sowie R die für die allgemeine Formel (1) genannten Bedeutungen besitzen.

7. Verbindung der Formel (2")

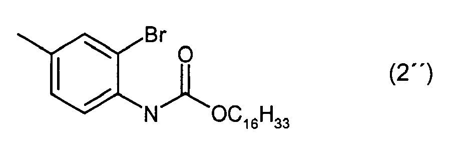

8. Verfahren zur Herstellung von 2-Alkyloxy-6-methyl-4*H*-3,1-benzoxazin-4-onen der allgemeinen Formel (11) 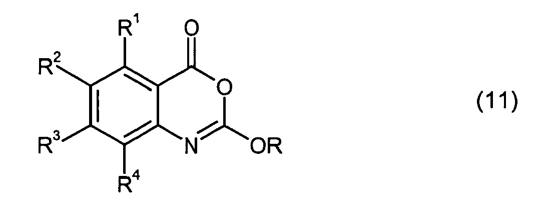 durch Umsetzung von Verbindungen der allgemeinen Formel (2) 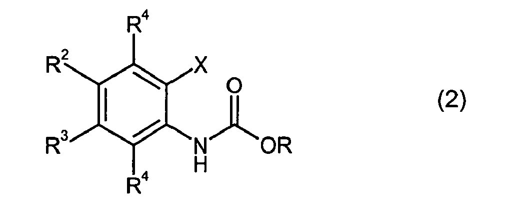 mit Kohlenmonoxid und Wasser in Gegenwart eines Palladiumkatalysators unter Bildung von Carbaminsäureester-Derivaten der allgemeinen Formel (1) 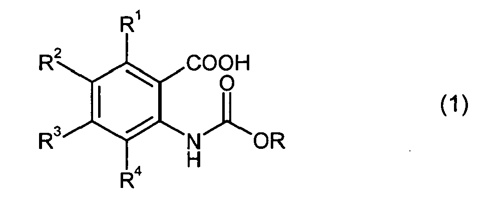 und deren Umsetzung mit Methyl- oder Ethylchlorformiat,
wobei R¹, R², R³, R⁴ sowie R in allen vorgenannten Formeln immer die für die allgemeine Formel (1) genannten Bedeutungen besitzen.

9. Verfahren nach Anspruch 8, **dadurch gekennzeichnet, dass** die Herstellung der Verbindungen der allgemeinen Formel (2) 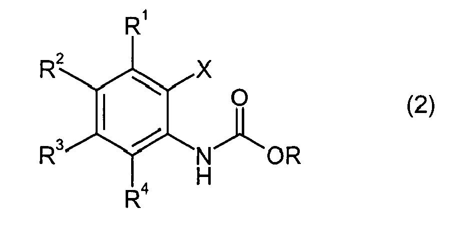 erfolgt, indem man ein gegebenenfalls substituiertes Anilin der allgemeinen Formel (6) 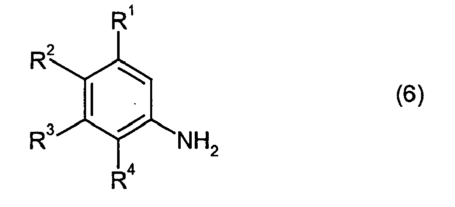 entweder
Ia) mit Phosgen umsetzt und
Ib) die dabei erhaltene Verbindung der allgemeinen Formel (7) 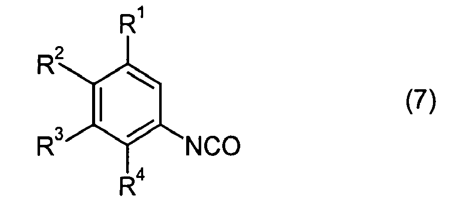 mit einem Alkohol der Formel ROH umsetzt
oder
II) mit einer Verbindung der Formel Cl-C(=O)OR umsetzt
und die sowohl nach Schritt (Ia) und (Ib) bzw. dem alternativen Schritt (II) erhaltene Verbindung der allgemeinen Formel (8) 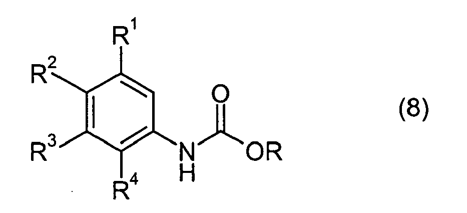 einer Halogenierung unter Bildung einer Verbindung der allgemeinen Formel (2) unterzieht, wobei in allen vorgenannten Formeln die Reste R¹, R², R³, R⁴ sowie R die für die allgemeine Formel (1) genannten Bedeutungen besitzen.

10. Verfahren nach Anspruch 8, **dadurch gekennzeichnet, dass** die Herstellung der Verbindungen der allgemeinen Formel (2) 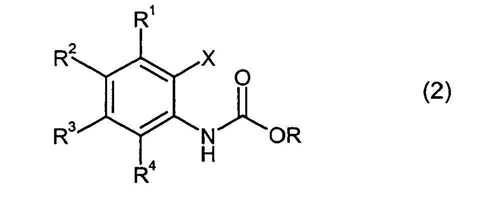 erfolgt, indem man ein gegebenenfalls substituiertes Anilin der allgemeinen Formel (6)
1) einer Halogenierung unterzieht und
2) die dabei erhaltene Verbindung der allgemeinen Formel (9) 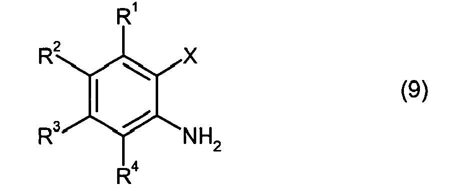 entweder
IIIa) mit Trichlormethylchlorformiat (Diphosgen) umsetzt und
IIIb) die dabei erhaltene Verbindung der Formel (10) 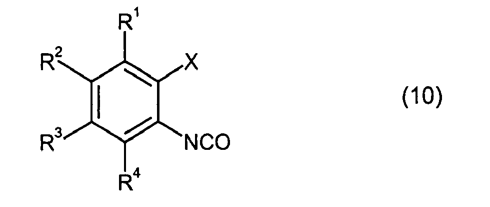 mit einem Alkohol der Formel ROH umsetzt
oder
IV) in einem einzigen Schritt mit Cl-C(=O)OR umsetzt,
wobei in allen vorgenannten Formeln die Reste R¹, R², R³, R⁴ sowie R die für die allgemeine Formel (1) genannten Bedeutungen besitzen.
